# EUROPEAN PATENT APPLICATION

(11) **EP 3 539 654 A1**
(43) Date of publication of application: **18.09.2019**
(21) Application number: 18382171.9
(22) Date of filing: 14.03.2018
(51) Int. Cl.: B01J 29/44, B01J 29/46, B01J 29/48, B01J 29/80, B01J 29/74, B01J 29/76, B01J 29/78, B01J 35/02, B01J 35/10, C07C 4/18, C07C 6/12

(54) **METHOD OF HEAVY REFORMATE CONVERSION INTO BTX OVER METAL-IMPREGNATED ZSM-5+NANOCRYSTALLINE BETA ZEOLITE COMPOSITE CATALYST; SAID COMPOSITE CATALYST**

(71) Applicant: Saudi Arabian Oil Company, Dhahran 31311 (SA); Instituto de Tecnologia Quimica (UPV-CSIC), 46022 Valencia (ES)
(72) Inventor: CORMA CANÓS, Avelino, 46022 Valencia (ES); PORTILLA OVEJERO, M. Teresa, 46022 Valencia (ES); MARTÍNEZ SÁNCHEZ, M. Cristina, 46022 Valencia (ES); NAVARRO VILLALBA, M. Teresa, 46022 Valencia (ES); SHAIKH, Sohel, 31311 Dhahran (SA); ABUDAWOUD, Raed Hasan, 31311 Dhahran (SA)
(74) Representative: Elzaburu S.L.P.

(57) **Abstract**

Method of making BTX compounds including benzene, toluene, and xylene, including feeding heavy reformate to a reactor containing a composite zeolite catalyst. The composite zeolite catalyst includes a mixture of nanocrystalline Beta zeolite (Nano-Beta) comprising crystal size in the range of 10 to 40 nm and ZSM-5. The Nano-Beta, the ZSM-5, or both include one or more impregnated metals. The method further includes producing the BTX compounds by simultaneously performing transalkylation and dealkylation of the heavy reformate in the reactor. The composite zeolite catalyst is able to simultaneously catalyze both the transalkylation and dealkylation reactions.

## Description

### Technical Field

Embodiments of the present specification generally relate to methods of making aromatic compounds, and specifically relate to methods of making benzene, toluene, and xylenes from a heavy reformate feed and a catalyst for utilization in the same.

### BACKGROUND

Heavy reformate (HR), containing mainly C₉₊ aromatics, is the fraction that remains after extraction of the more valuable BTX (benzene, toluene, xylene) fraction from the catalytic reformate or the pyrolysis gasoline. Traditionally this fraction was directly added to the gasoline pool. However, due to the restriction of the benzene content in gasoline by environmental regulations, it is important to find alternative ways of upgrading this stream into other valuable products. One option is to convert the heavy aromatics in the heavy reformate into additional BTX compounds. Heavy reformate may be converted into xylenes, benzene, and toluene by dealkylation of the C₉₊ alkylaromatics to produce toluene, and further transalkylation of the toluene formed by dealkylation with other C₉₊ alkylaromatics present in the feed to produce benzene and xylenes. Regardless, these means to produce BTX compounds by simultaneous dealkylation and transalkylation have limited efficiency, because of the sequential nature of the conversion reaction process where products of a first reaction are utilized in a second reaction.

### SUMMARY

Accordingly, ongoing needs exist for catalysts suitable for efficiently converting heavy reformates to produce benzene, toluene, and xylenes. Embodiments of the present disclosure are related to composite zeolite catalyst formulations and methods of making benzene, toluene, and xylenes using the composite zeolite catalyst. The composite zeolite catalysts may convert a mixture of heavy aromatic compounds (such as those present in heavy reformate), particularly C₉₊ aromatic hydrocarbons to benzene, toluene, and xylenes, and particularly to commercially valuable xylenes.

According to one embodiment, a method of making BTX compounds including benzene, toluene, and xylene is provided. The method includes feeding heavy reformate to a reactor. The reactor contains a composite zeolite catalyst including a mixture of nanocrystalline Beta zeolite (Nano-Beta) comprising crystal size in the range of 10 to 40 nanometers (nm) and ZSM-5. The Nano-Beta, the ZSM-5, or both include one or more impregnated metals. The method further includes producing the BTX compounds by simultaneously performing transalkylation and dealkylation of the heavy reformate in the reactor. The composite zeolite catalyst is able to simultaneously catalyze both the transalkylation and dealkylation reactions.

According to another embodiment, a composite zeolite catalyst is provided. The composite zeolite catalyst includes a mixture of nanocrystalline Beta zeolite (Nano-Beta) comprising crystal size in the range of 10 to 40 nm and ZSM-5. The Nano-Beta, the ZSM-5, or both comprise one or more impregnated metals.

Additional features and advantages of the described embodiments will be set forth in the detailed description which follows, and in part will be readily apparent to those skilled in the art from that description or recognized by practicing the described embodiments, including the detailed description which follows, the claims, as well as the appended drawings.

### BRIEF DESCRIPTION OF THE DRAWINGS

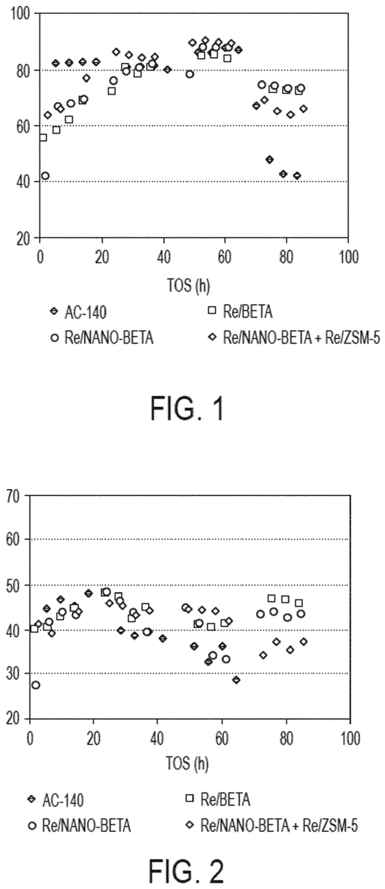

FIG. 1 is a graph of Methylethylbenzene (MEB) conversion of a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 2 is a graph of Trimethylbenzene (TMB) conversion of a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

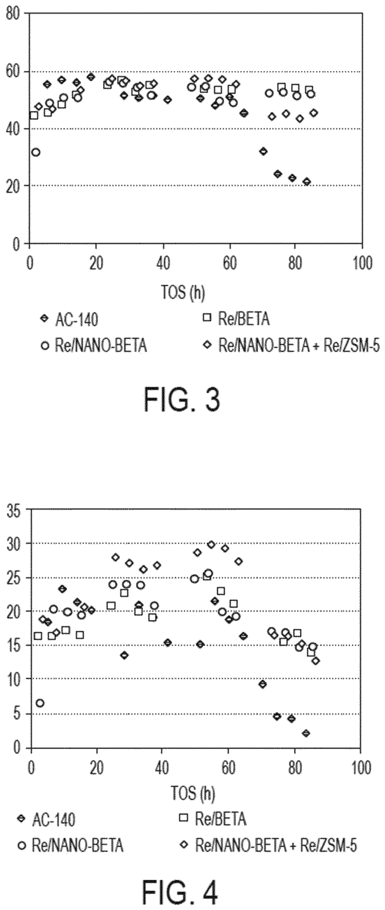

FIG. 3 is a graph of overall conversion (MEB+TMB) of a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 4 is a graph of xylenes yield from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

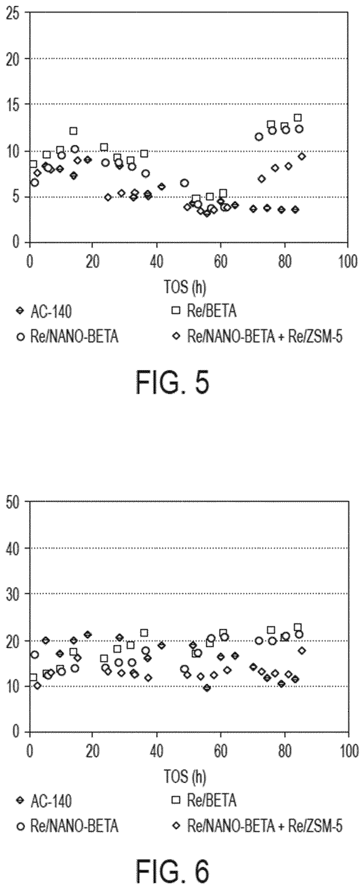

FIG. 5 is a graph of A₁₀ yield (yield of aromatics with 10 carbons) from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure..
FIG. 6 is a graph of A₁₀₊ yield (yield of aromatics with more than 10 carbons) from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

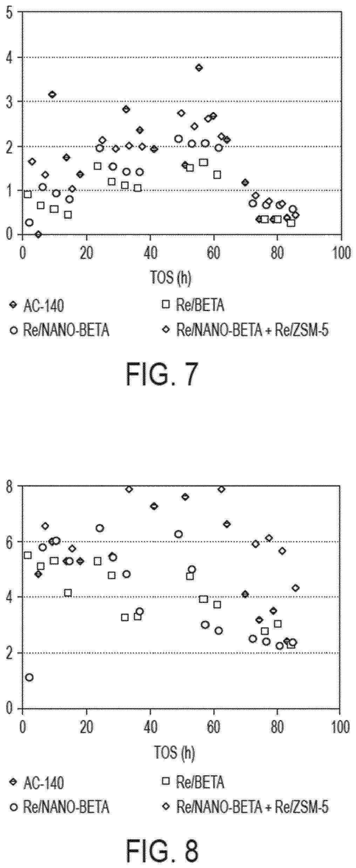

FIG. 7 is a graph of light hydrocarbon yield from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 8 is a graph of toluene yield from of a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

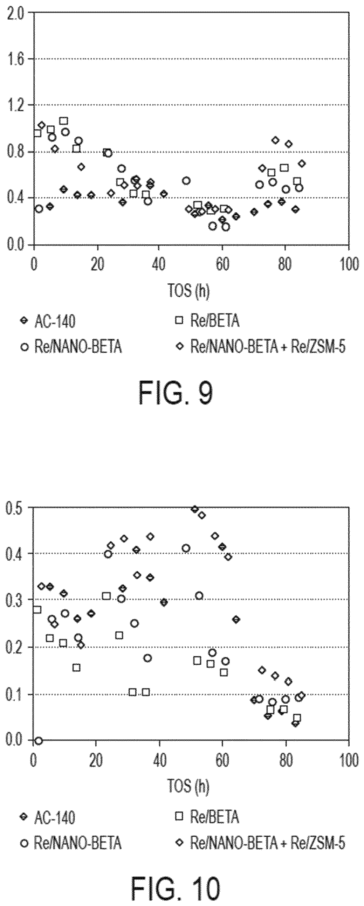

FIG. 9 is a graph of ethylbenzene yield from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 10 is a graph of benzene yield from a simulated heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

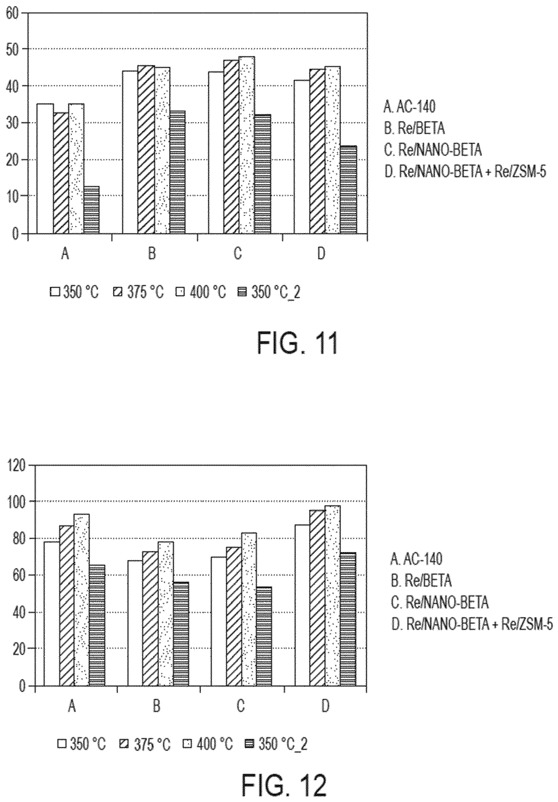

FIG. 11 is a graph of TMB conversion of an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 12 is a graph of MEB conversion of an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

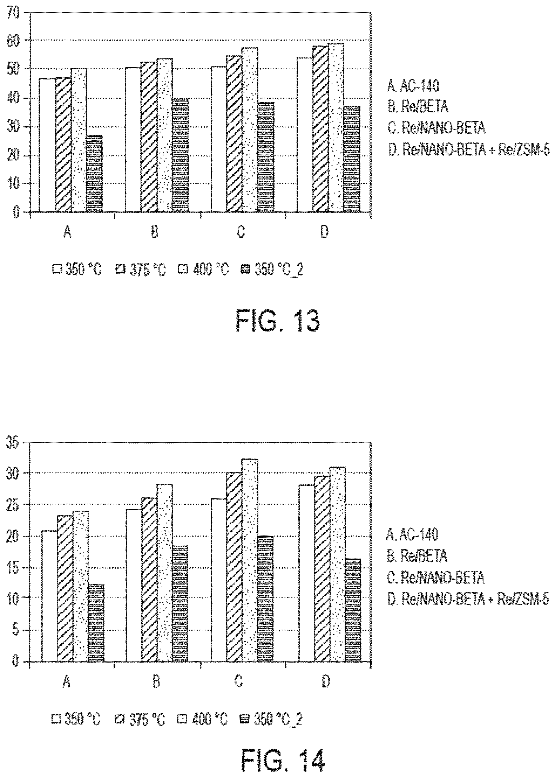

FIG. 13 is a graph of overall conversion of an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 14 is a graph of xylenes yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

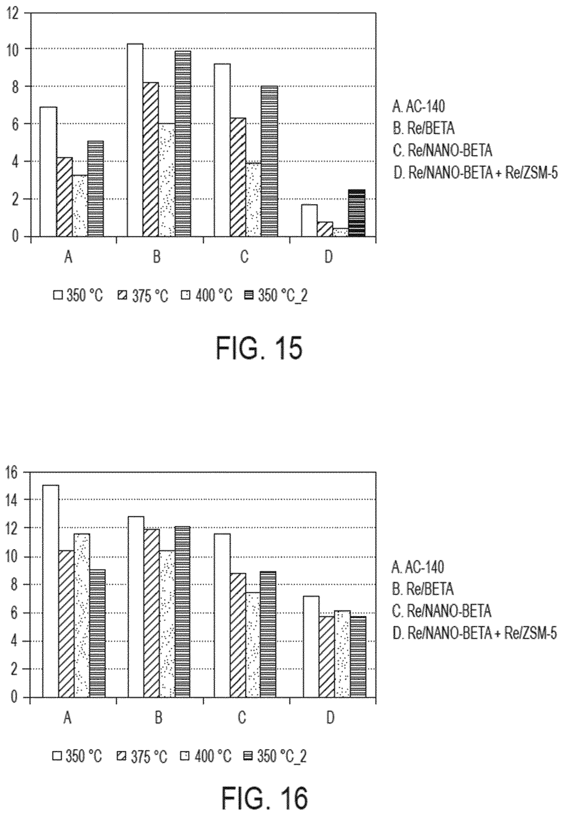

FIG. 15 is a graph of A₁₀ yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 16 is a graph of A₁₀₊ yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

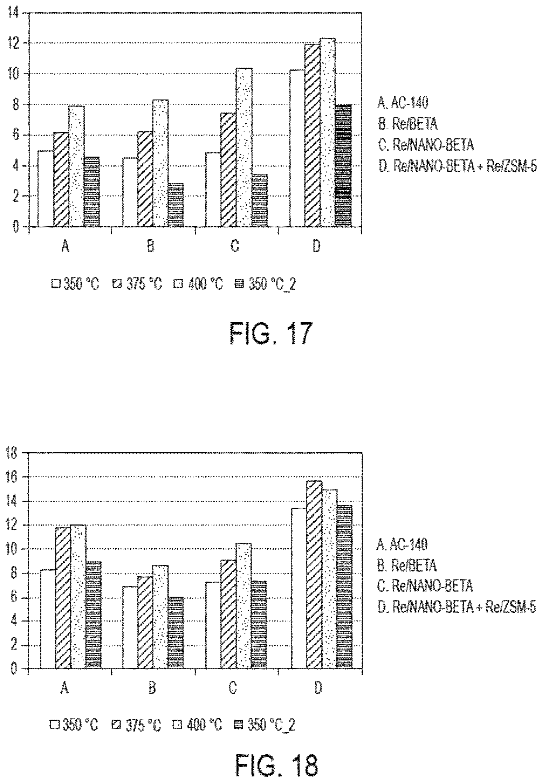

FIG. 17 is a graph of light hydrocarbon yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 18 is a graph of toluene yield from of an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

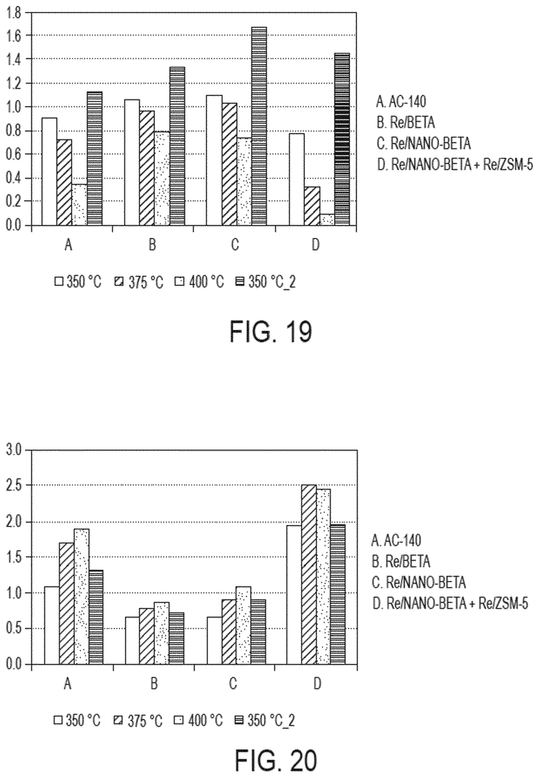

FIG. 19 is a graph of ethylbenzene yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.
FIG. 20 is a graph of benzene yield from an industrial heavy reformate stream obtained with commercially available zeolite catalysts, Nano-Beta impregnated with 0.36 weight percent (wt. %) rhenium, and a composite zeolite catalyst in accordance with one or more embodiments of the present disclosure.

### DETAILED DESCRIPTION

Reference will now be made in detail to embodiments of a method of making benzene, toluene, and xylene by conversion of heavy reformate with a composite zeolite catalyst.

The main components of heavy reformate are ethyl-toluenes (methyl-ethyl-benzenes, MEB) and trimethyl-benzenes (TMB). The structures of the MEB isomers and TMB isomers are provided *infra.*

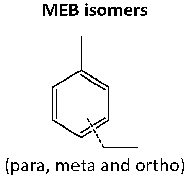

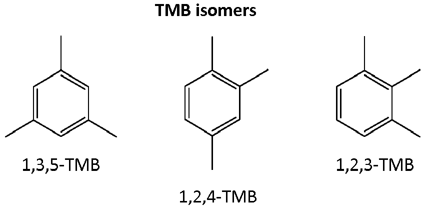

These aromatics can be converted into the more valuable BTX compounds by means of dealkylation of the C₉₊ alkylaromatics, or by transalkylation of these compounds with benzene or toluene. The aim of the process is to maximize the production of xylenes by de-ethylation of MEB and transalkylation of TMB. Specifically, transalkylation of TMB present in the feed with the toluene formed as a product of de-ethylation of MEB.

The dealkylation of MEB to toluene and ethane is provided *infra.* Dealkylation of MEB in the presence of a Brønsted acid catalyst initially produces toluene and ethylene. However, the ethylene may be subsequently hydrogenated to ethane in the presence of an adequate hydrogenation catalyst. If the hydrogenation functionality is not effective, portions of the ethylene may not be hydrogenated to ethane and as such may be present in the product gases, or it may be converted to oligomers or other products.

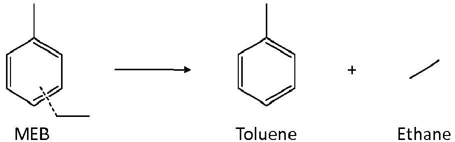

The transalkylation of TMB present in the heavy reformate with the toluene formed from dealkylation of MEB to toluene and ethane is provided *infra.*

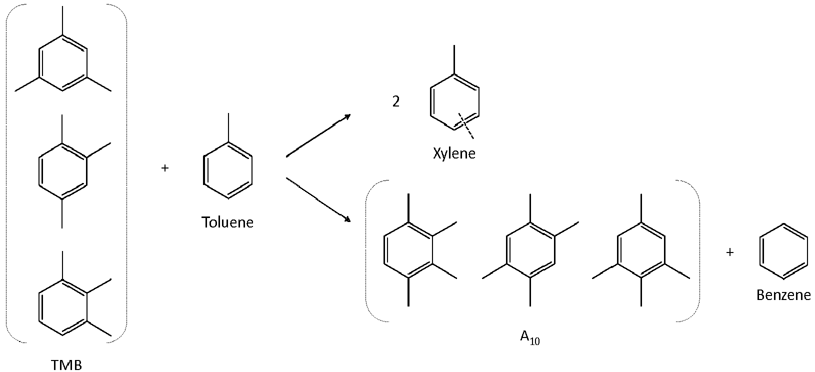

Additionally, toluene and TMB may also undergo a disproportionation reaction leading to xylenes and benzene or xylenes and tetramethylbenzenes (A₁₀), respectively. The chemical reactions are provided *infra.*

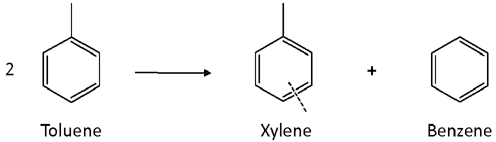

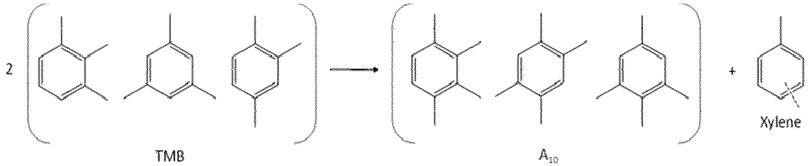

A composite zeolite catalyst may be formed from a mixture of nanocrystalline beta (Nano-Beta) and ZSM-5 zeolite catalysts. The composite zeolite catalyst allows conversion of heavy reformate, or other aromatic reactant streams, in a single reactor. Specifically, the dealkylation of MEB and the transalkylation of the produced toluene with TMB may be performed in a single reactor, because of the proximity between the crystals of Nano-Beta and ZSM-5 when physically mixed in a single reactor. The MEB dealkylation reaction is necessary in order to obtain the toluene that has to react with the TMB in the feed for producing the desired xylenes. Thus, the proximity of the ZSM-5 and Nano-Beta crystals obtained by the physical mixing of the Nano-Beta and ZSM-5 and reaction in a single reactor enables an improved and faster coupling of both consecutive reactions to produce benzene, toluene, and xylenes.

The composite zeolite catalyst in one or more embodiments comprises Nano-Beta and ZSM-5. ZSM-5 is an aluminosilicate zeolite of the pentasil family of zeolites. ZSM-5 (Zeolite Socony Mobil-5) has a Mordenite Framework Inverted (MFI) framework with an ordered crystal structure presenting intersecting 10-ring channels (5.1x5.5 and 5.3x5.6 angstrom (Å)). Zeolite beta, an aluminosilicate, comprises a molecular structure of a framework containing an intergrowth of two distinct structures termed Polymorphs A and B. The polymorphs grow as two-dimensional sheets and the sheets randomly alternate between the two. Both polymorphs have a three dimensional network of 12-ring pores. The nanocrystalline beta (Nano-Beta) has the same microporous crystalline structure as generic beta as well as the same chemical formulation and composition. However, Nano-Beta presents differing textural properties characterized by a comparatively larger external surface area, especially a larger mesopore volume, due to the presence of crystallites with sizes ranging from 10 to 40 nanometers (nm).

The method of making BTX compounds includes feeding heavy reformate to a reactor, which contains the composite zeolite catalyst formed from a mixture of Nano-Beta and ZSM-5. The BTX compounds are then produced by simultaneously performing transalkylation and dealkylation of the heavy reformate in the reactor. The composite zeolite catalyst is able to simultaneously catalyze both the transalkylation and dealkylation reaction with the combination of Nano-Beta and ZSM-5.

The composite zeolite catalyst including both Nano-Beta and ZSM-5 produces a synergistic effect and is capable of converting a heavy reformate feed, generally comprising at least 15 weight percent (wt. %) MEB and at least 50 wt. % TMB, to BTX compounds within a single reactor. The presence of ZSM-5 component increases the amount of toluene in the reaction media as a result of the additional dealkylation activity originating from the ZSM-5 component. The increased availability of toluene in the reaction media allows for increased reaction with the TMB present in the heavy reformate feed in conjunction with the Nano-Beta to yield BTX products, especially desirable xylenes. Additionally, as the TMB has more toluene available for reaction, transalkylation of the A₉₊ aromatics from the heavy reformate feed is reduced, which also reduces the generation of undesirable A₁₀₊ by-products.

Alkylaromatics, such as those present in a heavy reformate (MEB, TMB), in the presence of an acid catalyst, may undergo undesired reactions, which lead to formation of aromatics with 10 carbon atoms (A₁₀) or more than 10 carbon atoms (A₁₀₊). If these A₁₀ and A₁₀₊ compounds cannot diffuse out of the zeolite crystals through the pores of the crystalline structure because of steric limitations, they may block part of the channel systems, lead to bulkier coke precursors, or both. The improved conversion efficiency of the composite zeolite catalysts alleviates the formation of heavy alkylaromatics comprising A₁₀ and A₁₀₊ aromatics. Specifically, the proximity of the ZSM-5 and Nano-Beta as a mixture within a single reactor allows the TMB of the feed to react preferentially with the toluene formed by dealkylation of MEB on the ZSM-5 crystals, instead of reacting with other TMB by transalkylation to form tetramethylbenzene or heavier compounds. The reaction of the heavy reformate feed in a single reactor in the presence of the composite zeolite catalyst, including ZSM-5 and Nano-Beta, results in higher selectivity to xylenes and reduced formation of A₁₀₊ and coke precursors, leading therefore to improved catalyst life.

The reaction of the heavy reformate feed in a single reactor with the composite zeolite catalyst comprising ZSM-5 and Nano-Beta achieves improved performance in conversion of the heavy reformate to BTX compounds. This improvement is even more profound when carrying out the transalkylation of a heavy reformate in the absence of added toluene or benzene, because these two aromatics must be produced *in-situ* from C₉₊ aromatics such as with dealkylation of MEB contained within the feed. The proximate locations of the Nano-Beta and ZSM-5 in the composite zeolite catalyst through a physical mixture and reaction in a single reactor allows the toluene produced from dealkylation of MEB to be more readily available for use in the transalkylation reaction of TMB or disproportionation reaction of toluene for the ultimate production of xylenes.

The Nano-Beta and ZSM-5 zeolite catalysts may be physically mixed in various ratios to produce a composite zeolite catalyst with varying degrees of MEB and TMB conversion. In one or more embodiments, the Nano-Beta and ZSM-5 are combined in a 50:50 to 90:10 weight ratio to form the composite zeolite catalyst. In various further embodiments, the Nano-Beta and ZSM-5 are combined in a 50:50 to 80:20 weight ratio, 50:50 to 70:30 weight ratio, 55:45 to 65:35 weight ratio, or an approximately 60:40 weight ratio to form the composite zeolite catalyst. As previously indicated, an increase in ZSM-5 in the composite zeolite catalyst results in an increase in the amount of toluene in the reaction media as a result of the additional dealkylation activity originated from the ZSM-5 component. However, it will be appreciated that an increase in ZSM-5 weight percentage (wt. %) requires a congruent reduction in Nano-Beta wt. %. An increase in ZSM-5 wt. % and reduction in Nano-Beta wt. % results in less transalkylation and an excess of toluene.

Moreover, the zeolite composite catalyst may be impregnated with metals for catalysis, for example, metals such as molybdenum, chromium, platinum, nickel, tungsten, palladium, ruthenium, gold, rhenium, rhodium, or combinations thereof. In one embodiment, the impregnated metal is rhenium (Re). The metal component may exist within the final zeolite composite catalyst as a compound, such as an active metal oxide, an active metal sulfide or active metal halide, in chemical combination with one or more of the other ingredients of the composite, or as an elemental metal. The impregnated metal component may be present in the final composite zeolite catalyst in any amount that is catalytically effective, for example from 0.01 to 20.0 wt. %, or from 2 to 5 wt. %, or from 0.1 to 1.5 wt. %, or from 0.3 to 0.5 wt. % of the composite zeolite catalyst particle.

Metals are added to the catalyst for their hydrogenation functionality. The dealkylation, transalkylation and disproportionation reactions take place on the Brønsted acid sites of the composite zeolite catalysts. However, the hydrogenation function of the metal component is utilized to convert ethylene into ethane and may also enhance the desorption of coke precursors. The conversion of ethylene into ethane avoids the oligomerization of the olefin to products that may deactivate the catalyst.

In one or more embodiments, the metals are incorporated into the catalyst by ion exchange or impregnation of their salts in aqueous solution. The catalysts with the incorporated metals are then calcined in air and the metals are converted into their oxide forms, which do not present hydrogenation activity. In order to be active for hydrogenation these oxides are converted into metal sulfides, for example metal sulfides of Mo, Ni, or W, or the metal oxides can be reduced to their elemental metal form, for example elemental forms of Mo, Pt, Re, Pd, or Rh. In one or more embodiments, the composite zeolite catalyst is impregnated with rhenium in the form of ammonium perrhenate (NH₄ReO₄) as a metal precursor through an incipient wetness procedure. In one or more embodiments, the composite zeolite catalyst is impregnated with molybdenum in the form of ammonium molybdate tetrahydrate ((NH₄)₆Mo₇O₂₄ · 4H₂O) as a metal precursor through an incipient wetness procedure.

In various embodiments, the impregnated metal component may be present in only the Nano-Beta, only the ZSM-5, or both. For example, the impregnated metal component may be in the Nano-Beta or ZSM-5 individually from 0.01 to 20.0 wt. %, or from 2 to 5 wt. %, or from 0.1 to 1.5 wt. %, or from 0.25 to 0.5 wt. %, or from 0.3 to 0.5 wt. % of the Nano-Beta or ZSM-5.

In one embodiment, the molar ratio of silicon to aluminum (Si/Al) in the Nano-Beta is from 6:1 to 30:1. In further embodiments, the molar ratio of silicon to aluminum in the Nano-Beta is from 7:1 to 20:1, from 8:1 to 10:1, or from 7:2 to 9:2.

The composite zeolite catalyst and the Nano-Beta and ZSM-5 individual components comprise porosity. A micropore volume and a mesopore volume represent the specific volumes corresponding to the microporous structure and to the mesoporous structure, respectively. The mesopores are mainly due to intercrystalline voids formed, because of the very small size of the zeolite crystals. The pore size ranges for mesopores and micropores are in conformity with conventionally understood size ranges for such pore classifications with micropores representing pores under 2 nanometers (nm) in diameter and mesopores representing pores of 2 to 50 nm in diameter. A total pore volume would additionally include any macropores if present.

From a property standpoint, in one or more embodiments, the Nano-Beta may have a micropore volume (V_{micro}) of at least 0.15 cubic centimeters per gram (cm³/g), or a micropore volume of at least 0.16 cm³/g, a micropore volume of 0.15 to 0.25 cm³/g, or a micropore volume of 0.16 to 0.20 cm³/g. The micropore volume may be calculated by the *t*-plot method of determining micropore volume known to one having skill in the art. Similarly, in one or more embodiments, the Nano-Beta may have a mesopore volume (Vₘₑₛₒ) of at least 0.30 cubic centimeters per gram (cm³/g), a mesopore volume of at least 0.35 cm³/g, a mesopore volume of 0.3 to 0.6 cm³/g, or a mesopore volume of 0.4 to 0.5 cm³/g. The mesopore volume may be calculated according to the Barrett-Joiner-Halenda (BJH) method of determining mesopore volume known to one having skill in the art. Details regarding the *t*-plot method and the BJH method of calculating micropore volume and mesopore volume respectively are provided in Galarneau et al., "Validity of the t-plot Method to Assess Microporosity in Hierarchical Micro/Mesoporous Materials", Langmuir 2014, 30, 13266-13274, for example. It is noted that the Nano-Beta has a higher Vₘₑₛₒ than commercial Beta (CP811) indicative of a smaller crystal size resulting in a larger ratio of external to internal surface area.

Regarding the ZSM-5 porosity, the ZSM-5 may have a micropore volume (V_{micro}) of at least 0.05 cubic centimeters per gram (cm³/g), or a micropore volume of at least 0.10 cm³/g, a micropore volume of 0.05 to 0.25 cm³/g, or a micropore volume of 0.10 to 0.20 cm³/g in accordance with the *t*-plot method of determining micropore volume. Similarly, in one or more embodiments, the ZSM-5 may have a mesopore volume (Vₘₑₛₒ) of at least 0.01 cubic centimeters per gram (cm³/g), or a mesopore volume of at least 0.03 cm³/g, a mesopore volume of 0.01 to 0.10 cm³/g, a mesopore volume of 0.03 to 0.08 cm³/g calculated according to the Barrett-Joiner-Halenda (BJH) method of determining mesopore volume.

The surface area of the pores of the composite zeolite catalyst and the Nano-Beta and ZSM-5 individually affect the TMB and MEB conversion of the heavy reformate. An increased surface area provides increased interaction between the individual catalyst components and the constituents of the heavy reformate feed allowing for increased conversion activity.

In one or more embodiments, the Nano-Beta may have a surface area defined by a Brunauer-Emmett-Teller (BET) analysis (S_{BET}) of at least 450 square meters per gram (m²/g), a S_{BET} surface area of at least 500 m²/g, a S_{BET} surface area of at least 550 m²/g, or a S_{BET} surface area of 575 m²/g to 650 m²/g. Further, Nano-Beta may have a micropore surface area (S_{micro}) of 325 m²/g to 425 m²/g. The micropore surface area may be calculated directly from the micropore volume. Additionally, the Nano-Beta may have an external surface area (S_{Ext}) of at least 150 m²/g, and preferentially, it may have an external surface area of 200 m²/g to 250 m²/g. It is noted that the external surface area is obtained as the difference between the BET surface area and the micropore surface area. It is noted that the Nano-Beta has a higher S_{Ext} than commercial Beta (CP811) as the smaller particle size results in a larger ratio of external to internal surface area.

In one or more embodiments, the ZSM-5 may have a surface area defined by a Brunauer-Emmett-Teller (BET) analysis (S_{BET}) of at least 300 square meters per gram (m²/g), a S_{BET} surface area of at least 325 m²/g, or a S_{BET} surface area of at least 350 m²/g. Further, the ZSM-5 may have a micropore surface area (S_{micro}) of 275 m²/g to 400 m²/g. In one or more embodiments, the ZSM-5 zeolite catalyst is a commercially available ZSM-5. For example, the ZSM-5 may be CBV3024E from Zeolyst International (Conshohocken, Pennsylvania, USA).

Nano-Beta is not a commercially available zeolite catalyst and therefore must be synthesized for utilization in the composite zeolite catalyst. It will be appreciated that various synthesis procedures may be utilized to prepare the Nano-Beta for utilization in the composite zeolite catalyst. In one embodiment, the synthesis of the Nano-Beta comprises combining a silicon source and an organic structure directing agent, and an aluminum precursor in a reagent container to form a catalyst gel. The catalyst gel is then heated to form the composite zeolite catalyst particles. One example synthesis procedure is detailed in the Examples section of this disclosure.

### EXAMPLES

The described embodiments will be further clarified by the following examples and comparative examples.

The following synthesis procedure for Nano-Beta serves as an example and differing synthesis procedures may equally be utilized to prepare the Nano-Beta for utilization in the composite zeolite catalyst.

For demonstration purposes, composite zeolite catalysts and the constituents of the composite zeolite catalysts were prepared in accordance with one or more embodiments of this disclosure. Composite zeolite catalyst particles were synthesized with rhenium incorporated into the catalyst particles. Rhenium was incorporated into the samples at 0.3 to 0.4 wt. % by means of the incipient wetness procedure using ammonium perrhenate (NH₄ReO₄) as a metal precursor. The rhenium was incorporated into the Nano-Beta and ZSM-5 individually and the generated zeolites were then physically mixed in a 60:40 Nano-Beta:ZSM-5 weight ratio to generate Example 1.

To synthesize Nano-Beta, 0.704 g. of Al(OH)₃, 27.14 g. of tetraethylammonium hydroxide (TEAOH) solution (35 wt. % in H₂O), and 9.4 g. of deionized water were mixed and stirred for approximately 15 minutes (min). To the resulting mixture, 6 g. of silica (SiO₂) were added and further stirred for 1 hour (h) at room temperature of approximately 20°C to 25°C to obtain a homogenous gel. The resulting homogenous gel was transferred into an autoclave lined with polytetrafluoroethylene (Teflon®) and kept in the autoclave at 140°C in static conditions for approximately 3 days. The gel was recovered from the autoclave and 4.5 g. of cetyltrimethylammonium bromide (CTABr) was added to the gel. The resulting mixture was transferred back into the autoclave and kept at 140°C at static conditions for an additional 7 days for crystallization. After filtration, in a final step, the resultant solid material from the autoclave was filtered and washed with deionized water, and dried in an oven at 100°C overnight. To eliminate residual organic material, the filtered and washed catalyst was calcined in static air at 580°C.

For demonstration purposes, a composite zeolite catalyst was prepared in accordance with one or more embodiments of this disclosure. The composite zeolite catalyst was formed as a physical mixture of rhenium containing Nano-Beta and rhenium containing ZSM-5. Pure Nano-Beta and pure ZSM-5 zeolites were impregnated with rhenium. Rhenium was incorporated into each sample at 0.3 to 0.4 wt. % by means of the incipient wetness procedure using ammonium perrhenate (NH₄ReO₄) as a metal precursor. The rhenium loaded Nano-Beta and ZSM-5 were mixed in a 60:40 weight ratio of Nano-Beta:ZSM-5. The ZSM-5 was the commercially available CBV3024E (Zeolyst International). The physical mixture was calcined in a fixed bed reactor with the temperature increased up to 500°C in a flow of nitrogen gas (N₂) at 100 milliliter per minute (ml/min) and then maintained at 500°C for 3 hours under air flow at 100 ml/min. The sample was then cooled to room temperature or to the reduction temperature under a nitrogen flow at 100 ml/min. The prepared composite zeolite catalyst with a 60:40 weight ratio of Nano-Beta with rhenium and ZSM-5 with rhenium is designated as Example 1 (Re/Nano-Beta+Re/ZSM-5).

Comparative zeolite catalyst samples were also prepared for comparison with the composite zeolite catalyst particles. Rhenium impregnated commercially available Beta (CP811 from Zeolyst International) was designated as Comparative Example 2 (Re/Beta) and the rhenium impregnated Nano-Beta was designated as Comparative Example 3 (Re/Nano-Beta). AC-140 (Comparative Example 4) represents a commercial catalyst based on a physical mixture of 60 wt. % PtNi/Beta, 20 wt. % ZSM-5, and 20 wt. % Ce/AP-3. The commercially available ZSM-5 (CBV3024E from Zeolyst International) impregnated with rhenium was designated as Comparative Example 5 (Re/ZSM-5). Similarly, zeolite catalyst samples were prepared for comparison without rhenium impregnation. Pure commercially available Beta (CP811) was designated as Comparative Example 6, the pure Nano-Beta was designated as Comparative Example 7, and the pure commercially available ZSM-5 (CBV3024E) was designated as Comparative Example 8.

A listing of the composition of each Example is provided in Table 1.

**Table 1: Composition of each Example**

| **EXAMPLE** | **COMPOSITION** |
|---|---|
| Example 1 | 60 wt. % Nano-Beta with 0.36 wt. % rhenium and 40 wt. % ZSM-5 with 0.35 wt. % rhenium |
| Comparative Example 2 | CP811 (Beta) with 0.36 wt. % rhenium |
| Comparative Example 3 | Nano-Beta with 0.36 wt. % rhenium |
| Comparative Example 4 | AC-140 |
| Comparative Example 5 | CBV3024E (ZSM-5) with 0.35 wt. % rhenium |
| Comparative Example 6 | CP811 (Beta) |
| Comparative Example 7 | Nano-Beta |
| Comparative Example 8 | CBV3024E (ZSM-5) |

The physico-chemical properties of each of the samples were quantified. Specifically, the silicon to aluminum ratio as well as the final wt. % of Re in each sample was determined for each sample type. The final wt. % of Re in each sample was determined with Inductively Coupled Plasma Atomic Emission Spectroscopy (ICP-AES). ICP-AES Analysis is a technique that can determine concentrations of trace to major elements and can detect most elements in the periodic table. Results can be obtained with detection limits in the parts per billion range. The ICP source converts the atoms of the elements in the sample to ions. These ions are then separated and detected by the mass spectrometer. Additionally, the micropore volume and the mesopore volume were calculated according to the *t*-plot method and the BJH correlation method respectively. Further, the micropore surface area was calculated from the micropore volume, the total specific surface area was calculated in accordance with the Brunauer-Emmett-Teller method widely used for evaluating the surface area of porous and finely-divided materials, and the external surface area was calculated based on the difference between the total specific surface area and the micropore surface area. These physio-chemical properties are delineated in Table 2.

**Table 2: Chemical composition and textural properties of samples**

| **Sample** | **Si/Al** | **Re (wt.%)** | **S_{BET} (m²/g)** | **S _{micro} (m²/g)** | **S_{Ext} (m²/g)** | **V _{micro} (cm³/g)** | **Vₘₑₛₒ (cm³/g)** |
|---|---|---|---|---|---|---|---|
| Comp. Example 4 (AC-140) | --- | --- | 457 | 372 | 85 | 0.18 | 0.26 |
| Comp. Example 6 (Beta) | 13.0 | --- | 587 | 378 | 210 | 0.18 | 0.30 |
| Comp. Example 2 (Re/Beta) | 10.1 | 0.36 | 582 | 376 | 2016 | 0.18 | 0.27 |
| Comp. Example 7 (Nano-Beta) | 9.2 | --- | 587 | 353 | 233 | 0.17 | 0.42 |
| Comp. Example 3 (Re/Nano-Beta) | 8.7 | 0.36 | 622 | 400 | 223 | 0.19 | 0.42 |
| Comp. Example 8 (ZSM-5) | 13 | --- | 372 | 332 | 40 | 0.16 | 0.05 |
| Comp. Example 5 (Re/ZSM-5) | 13.6 | 0.35 | 371 | 331 | 39 | 0.16 | 0.05 |

Table 2 illustrates that the Nano-Beta and Beta have differing properties which may have a direct effect on the catalytic behavior of a composite zeolite catalyst formed from each one respectively. It is initially noted that the Si/Al molar ratio of Nano-Beta is lower than that of the Beta. Additionally, the Nano-Beta comprises a greater external surface area and a greater mesopore volume. The greater Sₑₓₜ and Vₘₑₛₒ is attributed to the small crystal size of Nano-Beta which results in a greater ratio of external to internal surface area of the formed crystals when compared to Beta.

The acidic properties of each of the samples were also quantified. Acidity measurements were carried out by adsorption/desorption of pyridine followed by IR spectroscopy. Self-supported wafers (10 milligrams per centimeter squared (mg cm⁻²)) of calcined samples, previously activated at 400°C and 10⁻² Pascal (Pa) overnight in a Pyrex vacuum cell, were allowed to come in contact with 6.5 × 10² Pa of pyridine vapor at room temperature and desorbed in vacuum at increasing temperatures (150°C, 250°C , and 350°C). The spectra were recorded at room temperature. All the spectra were scaled according to the sample weight. Brønsted and Lewis acidity of the samples compared are given in arbitrary units, according to the intensity of the bands assigned to the pyridine interacting with the Brønsted and Lewis acid sites of the zeolites (1550 and 1450 cm⁻¹, respectively). These acidic properties are listed in Table 3.

**Table 3: Acidic properties of samples**

| | **Brønsted Acidity (u.a.)** | | | | **Lewis Acidity (u.a.)** | | |
|---|---|---|---|---|---|---|---|
| **Sample** | **B150** | **B250** | **B350** | **B350/B 150** | **L150** | **L250** | **L350** |
| Comp. Example 4 (AC-140)* | 110 | 72 | 45 | 0.45 | 252 | 207 | 148 |
| Comp. Example 6 (Beta) | 218 | 162 | 90 | 0.55 | 512 | 486 | 413 |
| Comp. Example 2 (Re/Beta) | 176 | 110 | 75 | 0.43 | 349 | 268 | 178 |
| Comp. Example 7 (Nano-Beta) | 441 | 392 | 289 | 0.66 | 402 | 400 | 400 |
| Comp. Example 3 (Re/Nano-Beta) | 235 | 186 | 102 | 0.43 | 645 | 440 | 391 |
| Comp. Example 8 (ZSM-5) | 439 | 395 | 337 | 0.77 | 51 | 36 | 32 |
| Comp. Example 5 (Re/ZSM-5) | 449 | 442 | 380 | 0.85 | 51 | 21 | 18 |

| | | | | | | | |
|---|---|---|---|---|---|---|---|
| * Acidity values normalized to zeolite content | | | | | | | |

Table 3 illustrates that Beta (Comparative Example 6) presents a lower Brønsted acid density and higher number of Lewis acid sites in comparison with Nano-Beta (Comparative Example 7). The difference in Brønsted acid site density and number of Lewis acid sites may be attributed to the lower dealumination degree of the Nano-Beta compared to the Beta. The differences in Brønsted acid site density is reduced after metal incorporation of rhenium. Further, the number of Lewis acid sites of rhenium incorporated Nano-Beta (Comparative Example 3) outnumbers those of rhenium incorporated Beta (Combative Example 2). It is further noted that the ZSM-5 samples (Comparative Examples 5 and 8) exhibit a high number of total Brønsted acid site density and have a high proportion of sites with higher strength, able to retain pyridine at the highest desorption temperatures (T=350°C, B350) with a significantly lower number of Lewis acid sites compared to the beta zeolites. Finally, the lower acidity values of AC-140 (Comparative Example 4) are noted when compared to the beta zeolites.

As stated previously, the present composite zeolite catalyst represents a dealkylation and transalkylation catalyst suitable for converting C₉₊ alkyl aromatic hydrocarbons to a product stream comprising benzene, toluene, and xylenes, particularly to commercially valuable xylenes. The feed stream to the conversion process generally comprises alkylaromatic hydrocarbons in the carbon number range C₉ to C₁₁₊ that may include, for example, such hydrocarbons as propylbenzenes, methylethylbenzenes, tetramethylbenzenes, ethyldimethylbenzenes, diethylbenzenes, methylpropylbenzenes, and mixtures thereof. For purposes of testing and quantifying the examples and comparative examples a simulated heavy reformate feed was generated. The simulated heavy reformate feed comprises 30 wt. % *para*-methylethylbenzene (*p*-MEB) and 70 wt. % 1,2,4-trimethylbenzene (1,2,4-TMB).

Catalytic test for conversion of the simulated heavy reformate feed were performed in a reaction system comprising sixteen (16) continuous fixed-bed parallel microreactors. Each reactor was capable of being fed independently with the desired flow of the simulated reformate feed and H₂ making it possible to operate in a wide range of contact times and hydrogen/hydrocarbon molar ratios. The simultaneous catalytic experiments were carried out under the following conditions: 20 bar total pressure, a hydrogen/hydrocarbon molar ratio of 8.5, a reaction time of 16 hours per temperature, and a weight hourly space velocity (WHSV) of 10 inverse hours (h⁻¹). After the testing at each temperature the zeolitic catalysts were kept at that temperature and under H₂ atmosphere for an additional 10 hours. Each zeolitic catalyst sample was prepared to a particle size of 0.2 to 0.4 millimeters (mm). The tested zeolitic samples included Example 1 (60:40 weight ratio Nano-Beta:ZSM-5 with 0.36 wt. % rhenium), Comparative Example 2 (commercially available Beta CP811 with 0.36 wt. % rhenium), Comparative Example 3 (Nano-Beta with 0.36 wt. % rhenium), and Compatative Example 4 (AC-140 based on a physical mixture of 60 wt. % PtNi/Beta, 20 wt. % ZSM-5, and 20 wt. % Ce/AP-3). Comparative Examples 2 and 3 serve to provide a comparison between commercially available Beta and Nano-Beta. Comparative Example 4 serves as a comparison with a commercially available composite zeolite composite.

Each fixed-bed microreactor reactor was prepared with 125 mg of the zeolitic catalyst sample and diluted with silicon carbide (SiC) to a total bed volume of 2.0 ml for testing. The experiments were performed on the same zeolite weight basis so in Comparative Example 4 (AC-140) the amount of catalyst added was adjusted according to its zeolite content in order to have 125 mg of zeolite excluding the matrix. Four consecutive reactions phases were completed at temperatures of 350°C, 375°C, 400°C, and a return to 350°C. After the testing at each temperature the zeolitic catalysts were kept at that temperature and under H₂ atmosphere for an additional 10 hours period.

The reaction products from each of the fixed-bed microreactors were analyzed by on-line gas chromatography using two independent channels (Bruker 450 Gas Chromatograph). Argon (Ar) as an internal standard, H₂, methane, and ethane were analyzed in a first channel equipped with a thermal conductivity detector (TCD) and three columns. The three columns were a Hayesep N pre-column (0.5 m length) (Hayes Separations, Inc.), a Hayesep Q (1.5 m length) (Hayes Separations, Inc.), and a 13X molecular sieve (1.2 m length). In a second channel the C₁-C₄ hydrocarbons were first separated from the aromatics in a CP-Wax capillary column (5.0 m length and 0.32 mm inner diameter) (Cole-Parmer). Subsequently, the C₁-C₄ gases were separated in a column with CP-PoraBOND Q (25 m length and 0.32 mm inner diameter) (Cole-Parmer) and detected in a flame ionization detector (FID). Separation of the aromatics was completed in a second CP-Wax (1.0 m length and 0.32 mm inner diameter) connected to a second FID.

With reference to FIGS. 1, 2, and 3, the MEB conversion (dealkylation), TMB conversion (transalkylation), and overall conversion (MEB+TMB) are illustrated for each of Example 1, Comparative Example 2, Comparative Example 3, and Comparative Example 4 versus time on stream (TOS). It is noted that the Nano-Beta based catalysts are more active than Comparative Example 4 and they did not exhibit deactivation during the testing procedure. This phenomenon is indicated by the conversion percentage for the initial 350°C stage at the beginning of each test and the final 350°C stage at the conclusion of each test being similar. Example 1 (60:40 Nano-Beta with 0.36 wt. % rhenium and ZSM-5 with 0.35 wt. % rhenium) and Comparative Example 3 (Nano-Beta with 0.36 wt. % rhenium) are highly active for conversion of TMB, and TMB+MEB respectively, and the conversion obtained is comparable, if not higher (at 400°C) than that of Comparative Example 2 (Beta with 0.36 wt. % rhenium) and Comparative Example 4 (AC-140). Mixing Nano-Beta with a medium pore ZSM-5 component decreases slightly the transalkylation capacity of the overall catalysts, but overall conversion is maintained or increased (see T=400°C) as compared to Nano-Beta.

The lack of deactivation observed for the composite zeolite catalyst is believed to be due to its higher catalytic efficiency, which reduces the formation of heavy alkylaromatics. The proximity of the two zeolite phases (ZSM-5 and Nano-Beta) because of the physical mixture of the two zeolite phases in a single catalyst and reactor allows the TMB present in the feed to preferentially react on the Nano-Beta crystals with the toluene previously formed by dealkylation of MEB on the ZSM-5 crystals. In fact, the overall conversion obtained with Example 1 is higher at 400°C than the one obtained with Comparative Examples 2 and 3, based on pure Beta and Nano-Beta respectively with no ZSM-5, due to a lower in-situ production of toluene. Additionally, the small crystal size of the Nano-Beta crystals creates short diffusion pathways which allow the products to diffuse out of the zeolite crystals before undergoing reactions into heavier aromatics, coke precursors, or both. This reduced formation of A₁₀₊ and coke precursors leads to improved catalyst life.

With reference to FIGS. 4, 5, 6, 7, 8, 9, and 10, the xylenes yield, A₁₀ yield, A₁₀₊ yield, light hydrocarbon yield, toluene yield, ethylbenzene yield, and benzene yield are respectively illustrated for each of the 4 sample types versus TOS. It is noted that Example 1 (60:40 Nano-Beta with 0.36 wt. % rhenium and ZSM-5 with 0.35 wt. % rhenium) favors the xylenes production at 375°C and 400°C as compared to Comparative Example 2 (pure Beta with 0.36 wt. % rhenium), Comparative Example 3 (pure Nano-Beta with 0.36 wt. % rhenium), and Comparative Example 4 (AC-140). The higher selectivity to xylenes is believed a consequence of the lower production of undesirable A₁₀₊ aromatics. As indicated in FIG. 6, Example 1 (60:40 Nano-Beta with 0.36 wt. % rhenium and ZSM-5 with 0.35 wt. % rhenium) demonstrated the lowest yield of A₁₀₊ aromatics at 375°C and 400°C.

The results generated from testing the samples with the simulated heavy reformate provided information regarding the relative activity of the different catalyst compositions and their stability towards deactivation with an extended TOS. The catalysts were also tested under conditions closer to industrial conditions which would be observed for conversion of heavy reformate to xylenes. To more accurately reflect industrial conditions a supply of actual industrial heavy reformate with known composition was utilized. Table 4 delineates the composition of the industrial heavy reformate used for testing and Table 5 provides the relative ratios of various components.

**Table 4: Industrial Heavy Reformate Composition**

| **Component** | | | **Mass %** |
|---|---|---|---|
| **Hydrocarbon Type** | **Hydrocarbon Sub-Type** | | |
| A₈ | Total | | 3.94 |
| | Ethylbenzene | | 0.03 |
| | *p*-xylene | | 0.15 |
| | *m*-xylene | | 0.38 |
| | *o*-xylene | | 3.38 |
| A₉ | Total | | 82.75 |
| | Isopropylbenzene | Total | 0.43 |
| | n-propylbenzene | Total | 2.07 |
| | Methylethylbenzene (MEB) | Total | 19.62 |
| | | *m*- and *p*-MEB | 15.33 |
| | | *o*-MEB | 4.29 |
| | Trimethylbenzene (TMB) | Total | 60.63 |
| | | 1,3,5-TMB | 11.69 |
| | | 1,2,4-TMB | 40.81 |
| | | 1,2,3-TMB | 8.13 |
| A₁₀₊ | Total | | 13.33 |

**Table 5: Industrial Heavy Reformate Composition**

| **Ratio** | | |
|---|---|---|
| **A₈** | | |
| | Ethylbenzene : Total A₈ | 0.0076 |
| | *p*-xylene : Total A₈ | 0.038 |
| | *m*-xylene : Total A₈ | 0.096 |
| | *o*-xylene : Total A₈ | 0.858 |

| **A₉** | | |
|---|---|---|
| | Isopropylbenzene : Total A₉ | 0.0052 |
| | n-propylbenzene : Total A₉ | 0.025 |
| | Total Methylethylbenzene (MEB) : Total A₉ | 0.237 |
| | *m*- and *p*-MEB : Total A₉ | 0.185 |
| | *o*-MEB : Total A₉ | 0.052 |
| | *m*- and *p*-MEB : Total MEB | 0.781 |
| | *o*-MEB : Total MEB | 0.219 |
| | Total Trimethylbenzene (TMB) : Total A₉ | 0.733 |
| | 1,3,5-TMB : Total A₉ | 0.141 |
| | 1,2,4-TMB : Total A₉ | 0.493 |
| | 1,2,3-TMB : Total A₉ | 0.098 |
| | 1,3,5-TMB : Total TMB | 0.193 |
| | 1,2,4-TMB : Total TMB | 0.673 |
| | 1,2,3-TMB : Total TMB | 0.124 |
| | Total A₉ : Total A₁₀₊ | 6.21 |

Catalytic tests for conversion of the industrial heavy reformate feed were performed in a fixed-bed stainless-steel tubular reactor. The reactor had a 10.5 mm internal diameter and a 20 centimeter (cm) length. The catalytic experiments in the fixed-bed tubular reactor were carried out under the following conditions: 20 bar total pressure, a hydrogen/hydrocarbon molar ratio of 4:1, a reaction time of 7 or 5 hours at each reaction temperature, and a weight hourly space velocity (WHSV) of 10 h⁻¹. The reactor was charged with 0.75 grams (g) of catalyst with a particle size of 0.2 to 0.4 mm for each test. The tested zeolitic samples included Example 1 (60:40 Nano-Beta with 0.36 wt. % rhenium and ZSM-5 with 0.35 wt. % rhenium), Comparative Example 2 (commercially available Beta CP811 with 0.36 wt. % rhenium), Comparative Example 3 (Nano-Beta with 0.36 wt. % rhenium), and Comparative Example 4 (AC-140). The catalyst was diluted with SiC to bring the total volume up to a total bed volume of 5.0 ml. For Comparative Example 4 (AC-140), the amount of catalyst added was adjusted according to its zeolite content in order to have 0.75 g of zeolite (the matrix was excluded). Gaseous compounds (H₂, N₂) were fed into the system by mass flow meters via a vaporizer. Nitrogen was also fed into the system as an internal reference. The industrial heavy reformate was fed by means of a high performance liquid chromatography (HPLC) pump to the vaporizer. The vaporizer was operated at 300°C and provided a steady and non-pulsing flow of reactants to the reactor. Prior to commencing the catalytic test, the catalyst was reduced in situ at 450°C for 1 h under H₂ flow (50 ml/min) at atmospheric pressure. For the catalytic testing, four consecutive reactions phases were completed at temperatures of 350°C (7 h reaction), 375°C (5 h reaction), 400°C (5 h reaction), and a return to 350°C (5 h reaction).

During reaction, the effluent stream was analyzed on-line at intervals of 32 minutes (min) in a Scion 456 Gas Chromatograph equipped with two detection channels. Nitrogen (N₂) as an internal standard, H₂, methane, and ethane were analyzed in a first channel equipped with a TCD and three columns. The three columns were a Hayesep N pre-column (0.6 m length) (Hayes Separations, Inc.), a Hayesep Q (1.6 m length) (Hayes Separations, Inc.), and a 13X molecular sieve (1.2 m length). In a second channel the C₁-C₄ hydrocarbons were first separated from the aromatics in a CP-Wax capillary column (5.0 m length and 0.32 mm inner diameter) (Cole-Parmer). Subsequently, the C₁-C₄ gases were separated in a column with CP-PoraBOND Q (25 m length and 0.32 mm inner diameter) (Cole-Parmer) and detected in a flame ionization detector (FID). Separation of the aromatics was completed in a second CP-Wax (39.5 m length and 0.32 mm inner diameter) connected to a second FID.

With reference to FIGS. 12, 13, 14, the TMB conversion (transalkylation), MEB conversion (dealkylation), and overall conversion (MEB+TMB) are respectively illustrated for each of Example 1, Comparative Example 2, Comparative Example 3, Comparative Example 4. It is noted that Example 1 (60:40 Nano-Beta with 0.36 wt. % rhenium and ZSM-5 with 0.35 wt. % rhenium) demonstrated greater overall conversion than the Comparative Examples which is attributed to the greater TMB conversion activity. The individual TMB conversion percentages and MEB conversion percentages are provided in Table 6.

**Table 6: TMB, MEB, and Overall Conversion**

| **Catalyst** | **Temperature** | **TMB Conversion (%)** | **MEB Conversion (%)** | **Overall Conversion (%)** |
|---|---|---|---|---|
| Example 1 (Re/Nano-Beta+Re/ZSM-5) | 350°C | 41.56 | 88.15 | 54.07 |
| | 375°C | 44.52 | 95.51 | 58.21 |
| | 400°C | 45.33 | 97.81 | 59.42 |
| | 350°C (Return) | 23.95 | 72.98 | 37.11 |
| Comparative Example 2 (Re/Beta) | 350°C | 44.22 | 68.12 | 50.64 |
| | 375°C | 45.38 | 72.13 | 52.57 |
| | 400°C | 45.10 | 78.00 | 53.93 |
| | 350°C (Return) | 33.75 | 55.58 | 39.61 |
| Comparative Example 3 (Re/Nano-Beta) | 350°C | 44.08 | 70.05 | 51.06 |
| | 375°C | 46.98 | 75.37 | 54.60 |
| | 400°C | 48.25 | 83.25 | 57.65 |
| | 350°C (Return) | 32.44 | 53.95 | 38.22 |
| Comparative Example 4 (AC-140) | 350°C | 35.51 | 78.58 | 46.89 |
| | 375°C | 32.79 | 87.01 | 47.11 |
| | 400°C | 35.19 | 92.95 | 50.45 |
| | 350°C (Return) | 12.66 | 65.48 | 26.62 |

With reference to FIGS. 14, 15, 16, 17, 18, 19, and 20, the xylenes yield, A₁₀ yield, A₁₀₊, yield light hydrocarbon yield, toluene yield, ethylbenzene yield, and benzene yield are respectively illustrated for each of Example 1, Comparative Example 2, Comparative Example 3, and Comparative Example 4. It is noted, with reference to FIG. 14, that Example 1 favors the xylenes production for all reaction temperatures as compared to Comparative Examples 2 and 4 which do not comprise the Nano-Beta. As xylenes are the desirable product, the increased yield of xylenes with the composite zeolite catalyst comprising Nano-Beta and ZSM-5 is a positive result. The higher selectivity to xylenes is believed a consequence of the lower production of undesirable A₁₀₊ aromatics. As indicated in FIG. 16, Example 1 demonstrated the lowest yield of A₁₀₊ aromatics. Similarly, Example 1 also demonstrates higher yields of other desirable products including toluene as shown in FIG. 18, benzene as shown in FIG. 20, and light hydrocarbons as show in FIG. 17. The numerical values of the yield as a wt. % for each catalyst is provided in Table 7. This improvement in xylenes and other light hydrocarbon production and concurrent reduction in A₁₀₊ fraction illustrates the benefit of the methods of the present disclosure where Nano-Beta and ZSM-5 are in proximate contact in a single reactor during heavy reformate feed conversion. An additional advantage is that the conversion may be completed in a single reactor, thereby reducing production complexity.

**Table 7: Product Yields**

| **Catalyst** | **Temper ature** | **Xylenes Yield (wt.%)** | **A₁₀ Yield (wt.%)** | **A₁₀₊ Yield (wt.% )** | **Light HC Yield (wt.% )** | **Toluene Yield (wt.%)** | **Ethylbenzen e Yield (wt.%)** | **Benzen e Yield (wt.%)** |
|---|---|---|---|---|---|---|---|---|
| Example 1 (Re/Nano-Beta+Re/ZSM-5) | 350°C | 28.13 | 1.70 | 7.08 | 10.24 | 13.36 | 0.78 | 1.94 |
| | 375°C | 29.58 | 0.76 | 5.78 | 11.93 | 15.67 | 0.33 | 2.49 |
| | 400°C | 31.10 | 0.44 | 6.08 | 12.36 | 14.99 | 0.10 | 2.45 |
| | 350°C (Return) | 16.37 | 2.49 | 5.70 | 8.01 | 13.65 | 1.45 | 1.96 |
| Comparative Example 2 (Re/Beta) | 350°C | 24.36 | 10.24 | 12.83 | 4.50 | 6.82 | 1.06 | 0.65 |
| | 375°C | 26.13 | 8.24 | 11.90 | 6.26 | 7.74 | 0.96 | 0.77 |
| | 400°C | 28.17 | 5.98 | 10.38 | 8.29 | 8.64 | 0.79 | 0.88 |
| | 350°C (Return) | 18.47 | 9.91 | 12.19 | 2.76 | 5.99 | 1.33 | 0.73 |
| Comparative Example 3 (Re/Nano-Beta) | 350°C | 26.07 | 9.19 | 11.63 | 4.86 | 7.24 | 1.10 | 0.68 |
| | 375°C | 30.06 | 6.27 | 8.80 | 7.40 | 9.16 | 1.03 | 0.91 |
| | 400°C | 32.18 | 3.91 | 7.47 | 10.18 | 10.55 | 0.74 | 1.07 |
| | 350°C (Return) | 20.06 | 8.03 | 8.89 | 3.43 | 7.29 | 1.67 | 0.91 |
| Comparative Example 4 (AC-140) | 350°C | 20.94 | 6.91 | 15.08 | 4.96 | 8.34 | 0.90 | 1.08 |
| | 375°C | 23.28 | 4.20 | 10.49 | 6.16 | 11.83 | 0.71 | 1.69 |
| | 400°C | 23.96 | 3.30 | 11.59 | 7.91 | 12.01 | 0.34 | 1.91 |
| | 350°C (Return) | 12.36 | 5.13 | 9.06 | 4.58 | 9.00 | 1.13 | 1.33 |

It should be understood that the various aspects of the method of making BTX compounds including benzene, toluene, and xylene, and the composite zeolite catalyst utilized in the same are described and such aspects may be utilized in conjunction with various other aspects.

It should be apparent to those skilled in the art that various modifications and variations can be made to the described embodiments without departing from the spirit and scope of the claimed subject matter. Thus, it is intended that the specification cover the modifications and variations of the various described embodiments provided such modification and variations come within the scope of the appended claims and their equivalents.

Throughout this disclosure ranges are provided. It is envisioned that each discrete value encompassed by the ranges are also included. Additionally, the ranges which may be formed by each discrete value encompassed by the explicitly disclosed ranges are equally envisioned.

## Claims

1. A method of making BTX compounds including benzene, toluene, and xylene, the method comprising:
feeding heavy reformate to a reactor, the reactor containing a composite zeolite catalyst comprising a mixture of nanocrystalline Beta zeolite (Nano-Beta) comprising crystal size in the range of 10 to 40 nm and ZSM-5, where the Nano-Beta, the ZSM-5, or both comprise one or more impregnated metals; and
producing the BTX compounds by simultaneously performing transalkylation and dealkylation of the heavy reformate in the reactor, where the composite zeolite catalyst is able to simultaneously catalyze both the transalkylation and dealkylation reactions.

2. The method of claim 1, where the heavy reformate comprises at least 15 wt. % methylethylbenzene (MEB) and at least 50 wt. % trimethylbenzene (TMB).

3. The method of claims 1 or 2, where the Nano-Beta comprises an external surface area greater than 150 m²/g.

4. The method of any of claims 1 to 3, where the one or more impregnated metals are selected from the group consisting of molybdenum, chromium, platinum, nickel, tungsten, palladium, ruthenium, gold, rhenium, rhodium, or combinations thereof and their respective oxides.

5. The method of any of claims 1 to 3, where the one or more impregnated metals comprises rhenium.

6. The method of any of claims 1 to 5, where the Nano-Beta, the ZSM-5, or both the Nano-Beta and ZSM-5 comprise up to 20 wt. % of the one or more impregnated metals.

7. The method of any of claims 1 to 6, where the composite zeolite catalyst comprises a mixture of Nano-Beta and ZSM-5 in a 50:50 to 90:10 weight ratio.

8. The method of any of claims 1 to 7, where the Nano-Beta has a molar ratio of silicon to aluminum (Si/Al) from 6:1 to 12:1

9. A composite zeolite catalyst,
the composite zeolite catalyst comprising a mixture of nanocrystalline Beta zeolite (Nano-Beta) comprising crystal size in the range of 10 to 40 nm and ZSM-5, where the Nano-Beta, the ZSM-5, or both comprise one or more impregnated metals.

10. The composite zeolite catalyst of claim 9, where the Nano-Beta comprises an external surface area greater than 150 m²/g.

11. The composite zeolite catalyst of claims 9 or 10, where the one or more impregnated metals are selected from the group consisting of molybdenum, chromium, platinum, nickel, tungsten, palladium, ruthenium, gold, rhenium, rhodium, or combinations thereof and their respective oxides.

12. The composite zeolite catalyst of any of claims 9 to 11, where the one or more impregnated metals comprises rhenium.

13. The composite zeolite catalyst of any of claims 9 to 12, where the Nano-Beta, the ZSM-5, or both the Nano-Beta and ZSM-5 comprise up to 20 wt. % of the one or more impregnated metals.

14. The composite zeolite catalyst of any of claims 9 to 13, where the composite zeolite catalyst comprises a mixture of Nano-Beta and ZSM-5 in a 50:50 to 90:10 weight ratio.

15. The composite zeolite catalyst of any of claims 9 to 14, where the Nano-Beta has a molar ratio of silicon to aluminum (Si/Al) from 6:1 to 12:1.
